# EUROPEAN PATENT SPECIFICATION

(11) **EP 1 815 801 B1**
(45) Date of publication and mention of the grant of the patent: **14.10.2009**
(21) Application number: 07250493.9
(22) Date of filing: 07.02.2007
(51) Int. Cl.: A61B 17/00, A61B 1/005, A61M 25/01, A61F 5/00, A61B 17/28, A61B 17/32

(54) **An improved articulating surgical instrument**
Verbessertes bewegliches chirurgisches Instrument
Instrument chirurgical articulé amélioré

(30) Priority: 07.02.2006 US 352471
(43) Date of publication of application: 08.08.2007
(73) Proprietor: ETHICON ENDO-SURGERY, INC., Cincinnati, Ohio 45242 (US)
(72) Inventor: Hassler, William L., Jr., Cincinnati Ohio 45249 (US); Widenhouse, Christopher W., Cincinnati, Ohio 45140 (US)
(74) Representative: Tunstall, Christopher Stephen

(56) References cited:
- DE-A1- 4 243 715
- DE-A1- 19 608 809
- FR-A1- 2 713 492
- US-A- 4 353 358

## Description

**Field of the Invention**

The present invention has application in conventional endoscopic and open surgical instrumentation as well as application in robotic-assisted surgery. The present invention has even further relation to adjustable surgically implantable bands, such as gastric bands for the treatment of obesity.

**Background of the Invention**

The percentage of the world's population suffering from morbid obesity is steadily increasing. Severely obese persons are susceptible to increased risk of heart disease, stroke, diabetes, pulmonary disease, and accidents. Because of the effect of morbid obesity to the life of the patient, methods of treating morbid obesity are being researched.

Numerous non-operative therapies for morbid obesity have been tried with virtually no permanent success. Dietary counseling, behavior modification, wiring a patient's jaws shut, and pharmacological methods have all been tried, and failed to correct the condition. Mechanical apparatuses for insertion into the body through non-surgical means, such as the use of gastric balloons to fill the stomach have also been employed in the treatment of the condition. Such devices cannot be employed over a long term, however, as they often cause severe irritation, necessitating their periodic removal and hence interruption of treatment. Thus, the medical community has evolved surgical approaches for treatment of morbid obesity.

Most surgical procedures for treatment of morbid obesity may generally be classified as either being directed toward the prevention of absorption of food (malabsorption), or restriction of stomach to make the patient feel full (gastric restriction) The most common malabsorption and gastric restriction technique is the gastric bypass. In variations of this technique, the stomach is horizontally divided into two isolated pouches, with the upper pouch having a small food capacity. The upper pouch is connected to the small intestine, or jejunum, through a small stoma, which restricts the processing of food by the greatly reduced useable stomach. Since food bypass much of the intestines, the amount of absorption of food is greatly reduced.

There are many disadvantages to the above procedure. Typically the above mentioned procedure is performed in an open surgical environment. Current minimally invasive techniques are difficult for surgeons to master, and have many additional drawbacks. Also, there is a high level of patient uneasiness with the idea of such a drastic procedure which is not easily reversible. In addition, all malabsorption techniques carry ongoing risks and side effects to the patient, including malnutrition and dumping syndrome.

Consequently, many patients and physicians prefer to undergo a gastric restriction procedure for the treatment of morbid obesity. One of the most common procedures involves the implantation of an adjustable gastric band. Examples of an adjustable gastric band can be found in U.S. Patents 4,592,339 issued to Kuzmak; RE 36176 issued to Kuzmak; 5,226,429 issued to Kuzmak; 6,102,922 issued to Jacobson and 5,601,604 issued to Vincent.In accordance with current practice, a gastric band is operatively placed to encircle the stomach. This divides the stomach into two parts with a stoma in-between. An upper portion, or a pouch, which is relatively small, and a lower portion which is relatively large. The small partitioned portion of the stomach effectively becomes the patients new stomach, requiring very little food to make the patient feel full.

Once positioned around the stomach, the ends of the gastric band are fastened to one another and the band is held securely in place by folding a portion of the gastric wall over the band and closing the folded tissue with sutures placed therethrough thereby preventing the band from slipping and the encircled stoma from expanding.

However, positioning the band around the stomach is often difficult. The band needs to be placed around the stomach, including the posterior side which the physician has little access to. One commercially available product available for the physician to do this is the Goldfinger® sold by Ethicon Endo-Surgery, Inc., Cincinnati OH. The instrument basically comprises a straight shaft that has an end which starts out straight but can progressively curve by actuation of a device at the instruments proximal end. Similar devices are also shown in patent literature such as the device disclosed in U.S. Patent 5,467,763 issued to McMahon et al. on November 21, 1995.

When using such a device, the physician uses it first as a blunt dissector to make what is referred to as the retrogastric tunnel. This is a tunnel behind the stomach which goes through the connective tissue surrounding the stomach just below the gastro-esophageal junction. Thereafter, the band is connected to the distal end of the device with suture, and the above described device drives or pulls the band through the tunnel. A straight instrument would have difficulty doing this, but an articulating one which can curve can do the job nicely.

DE 19608809 discloses a device for aiding a surgical procedure comprising a steerable distal tip, said tip comprising a plurality of interconnected link elements.

However, there has been a desire to improve upon the commercially available articulating surgical devices.

**Summary of the Invention**

In accordance with the present invention there is provided a device for aiding a surgical procedure, the device having an elongated shaft with a distal end, a proximal end and a longitudinal axis therebetween. The proximal end has a handle attached thereto, and the distal end has an end effector attached thereto. The shaft has first and second spaced apart strips running along the longitudinal axis and attached to a distal end of the end effector. The end effector includes a plurality of spaced apart links disposed along the longitudinal axis between the distal end of the end effector the distal end of the shaft. The strips run through the links, and each link is attached to at least one strip. The device also includes an actuator attached to the handle. The actuator pulls the first strip proximally, causing the links and the end effector to curve in the direction of the first strip. The actuator also includes a means for preventing the first strip from moving proximally beyond a predetermined amount and thereby causing the end effector to bend only up to a predetermined angle. In addition, the predetermined angle is such that the links never make contact with one another.

**Detailed Description of the Drawings**

The novel features of the invention are set forth with particularity in the appended claims. The invention itself, however, both as to organization and methods of operation, together with further objects and advantages thereof, may best be understood by reference to the following description, taken in conjunction with the accompanying drawings in which:

Figure 1 is a plan view of device 10 made in accordance with the present invention.

Figure 2A is a perspective view an end effector for device 10 made in accordance with the present invention.

Figure 2B is a cross-section view of Figure 1, taken along lines 2B-2B.

Figure 3 is a close up view showing a portion of the end effector shown in Figure 2B

Figure 4 is another close up view showing a portion of the end effector shown in Figure 2B.

Figure 5 is a plan view of the end effector shown in Figure 2A.

Figure 6 is a cross-section view of the handle shown in Figure 1, taken along lines 6-6.

Figure 7 is a plan view of the end effector shown in its articulated state.

**Detailed Description of the Invention**

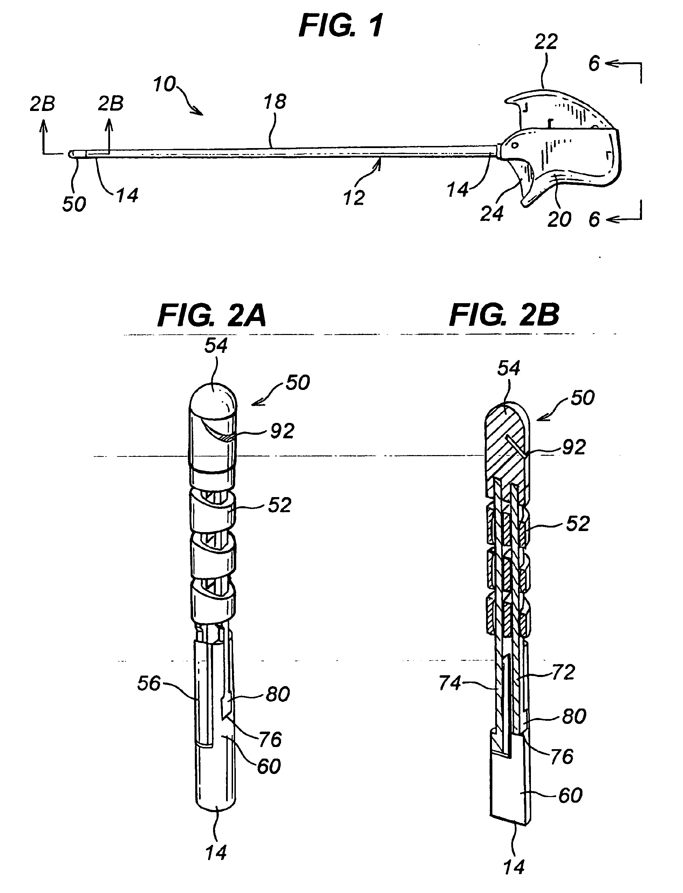

Referring now to the drawings wherein like numerals indicate the same element throughout the views, there is shown in figure 1 a device 10, for aiding in a surgical procedure. Device 10 has an elongated shaft 12 with a distal end 14, a proximal end 16 and a longitudinal axis 18 therebetween. The proximal end 16 has a handle 20 attached thereto, and the distal end 14 has an end effector 50 attached thereto. Handle 20 has an actuator 22, and an actuator release 24 attached thereto. As will be apparent later, the handle is held in the surgeon's hand with the thumb portion of the surgeon's palm resting over actuator 22. The angle that end effector 50 bends is controlled by the amount that the actuator is depressed. The more actuator 22 is depressed into the handle, the larger the angle of end effector articulation. However, before actuator 22 can be moved, the surgeon must release it by pressing the actuator release 24 into the instrument handle, and holding it there while the articulation angle is changed. In addition, to release the end effector articulation causing it to go straight, the release trigger must be depressed into the handle.

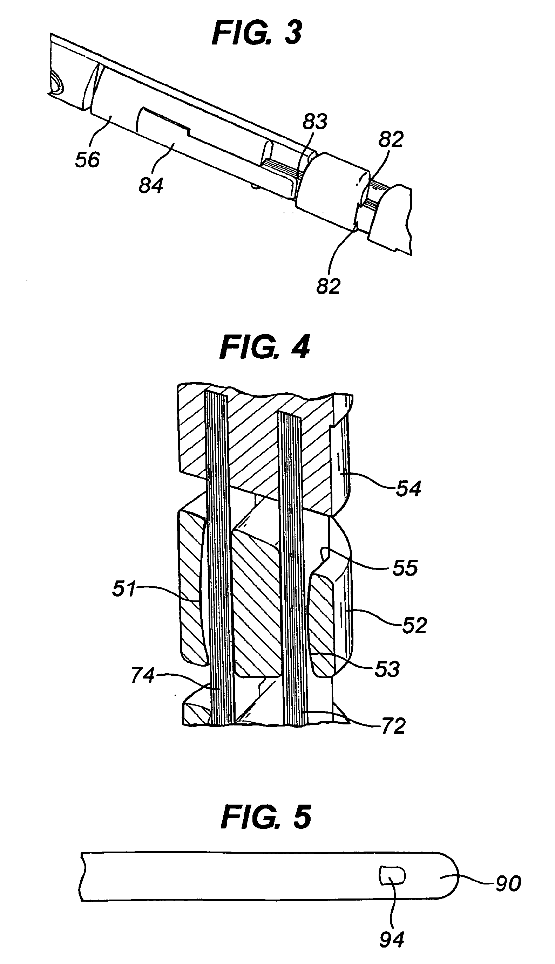

As seen from Figures 2A, 2B, and 3, the end effector includes a plurality of spaced apart links 52 disposed along the longitudinal axis between the distal end of the end effector 54 the distal end of the shaft 14. Shaft 12 has a first strip 72 running along the longitudinal axis and through links 52 from the handle 20 to a distal end of the end effector 54 where it is attached thereto. Device 10 also includes a second strip 74 running along the longitudinal axis through links 52 from the distal end 14 of shaft 12. where it is rigidly attached thereto, to distal end 54 of end effector 50. First and Second Strips are preferably made from stainless steel or any other suitable material known to those skilled in the art. In addition, strips 72 and 74 and can actually comprise a bundle or plurality of strips. Having a plurality of juxtapose strips provide for greater stiffness while still providing good bending. The Fixed base 56 holds the second strip or bundle of strips 74. Base 56 and strips 74 are is rigidly attached to the shaft 12. The Sliding base 60, which pulls on the first strip or plurality of strips 74 in a proximal direction, causes end effector 50 to bend or articulate in a direction towards first strip 72.

Both sets of strips 72 and 74 are attached to the distal end of end effector 54. Since the individual strips act as leaves in a leaf spring, they have to move in a linear direction with respect to each other. Because the leaves are rigidly fixed in the tip, they therefore have to slide with respect to each other within the pockets 76 of the bases 56 and 60 that hold them during actuation of the articulating tip. The larger the angle achieved during tip articulation, the more these strips will slide with respect to each other within the base pockets 76. These pockets have to be significantly longer than the length of the square tabs 80 located at the bottom of the strips being held. This provides adequate clearance needed for the full range of sliding motion of the strips with respect to each other.

Links 52 act as anti-buckling devices for the strips 72 and 74, which hold the two sets or bundles of strips 72 and 74 at a fixed distance from each other, and keep the sets of strips, or leaf springs from buckling. These links also keep the individual strips or leaves from buckling with respect to each other within the bundle so that they act more like a single beam in bending, reinforcing each other like a normal leaf spring. These links are made in two identical halves that will eventually be designed to be pressed fit into each other to hold the strips together, and form a single link.

Strips 72 in the movable base 60 can be covered by a thin Teflon, or other lubricious material that keeps them from popping out of the pocket 76, and helps reduce the friction of the base sliding against the inside of the instrument shaft. As seen from the figures, strip or strips 72 have a series of notches 82 to hold links 52 in place. Strip or strips 74 can be held in place by a stainless steel member or fixed base retainer 84 that extends beyond the end of the fixed base 56. Retainer 84 also retains the notched strips 74 in the pocket, and helps improve the stiffness of the tip.

As seen in figure 4, links 52 have an the internal concave contour 51 curved in the direction of articulation (concaved). The radius of curvature of contour feature 51 is less than the radius of curvature of strip 74 when it is bent to its maximum angle to help prevent binding. If the internal slots of the links were not curved in this fashion, then the strips would be forced into bending only between the links, and this would probably result in plastic deformation of the strips, at low force levels. Also, please note the radiused corners where the internal contour cavities intersect with both the top and bottom face of the link. This radius avoids constraining the strips during articulation.

The slots 55 in links 52 containing strips 72 has a contour 53 curvature (convexed). This curvature is to keep the link from pinching the strips 72 when they are being pulled through the link 52. The friction between the strips 72 and the link 52 during articulation will tend to cause the link to rotate slightly downwards which would tend to bind the links 52 and strips 72. By having this reverse curvature, this frictional binding is minimized, and its' effect on the force to actuate the articulating tip is also minimized. The larger this binding friction, the less round the articulation will be. Put another way, the higher the friction, the more bending will occur at the base of the tip, and less at the top. This might eventually require the use of a lubricant in the links such as Sodium Sterate.

As seen from figure 5, end effector 54 can be covered by a cylindrical sleeve, or cover 90 to prevent human tissue from catching on the links, and strips, resulting in tissue trauma. Cover 90 can be made from an elstomer such as silicone or a polyurethane thermoplastic elastomer, such as Pellethane (heat shrink tubing). End effector 50 acts as a blunt dissector when doing a surgical procedure such as those described in the background section herein. The dissection tip also has two different notch features. There is an angled notch 92 which can receive a suture loop on the end of an adjustable gastric band. There is also a keyhole feature 94 which can receive a band pulling tab present on many adjustable gastric bands.

The links 52 should also be as long as possible but also cannot be allowed to touch each other as the tip articulates. Causing them to touch may upset the shape the tip will take, as well as the uniformity of the force loading in the tip. It is also necessary that the links not touch each other even under load, as these links are not designed to take axial loads, only the anti-buckling loads that are small.

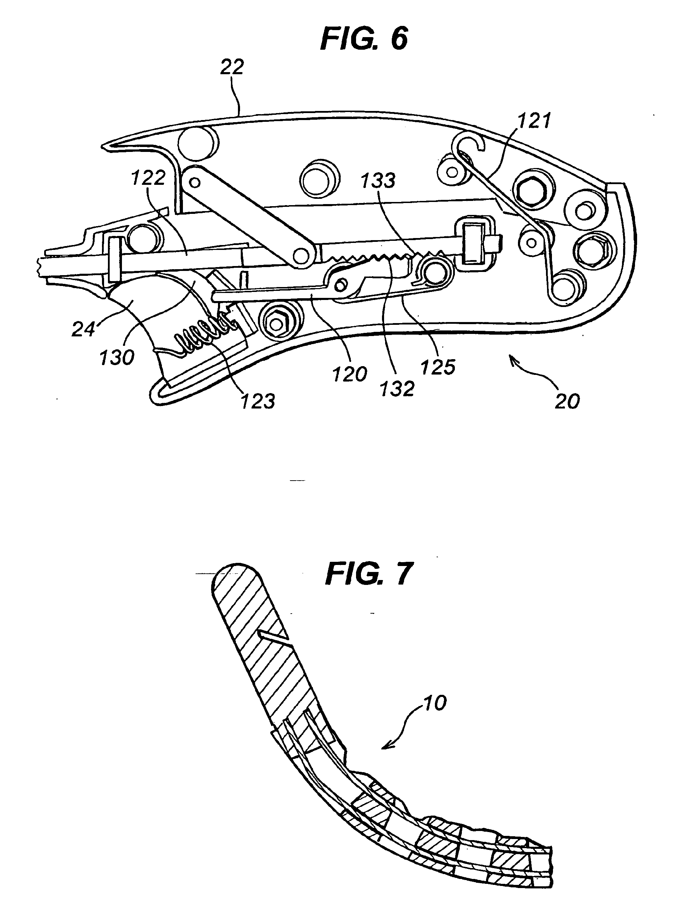

Referring now to Figure 6, there is shown the handle 20 of device 10. Handle 20 is very similar to the handle of the device described in U.S. Patent application serial no. 10/741875, publication no. US-2004-0254537-A1, which is hereby incorporated herein by reference. When the surgeon depresses the articulation release trigger 24 with his index finger, the shaft 130 on the back of the release trigger 24 pushes on the ratchet pall arm 120 causing it to rotate. This rotation causes the teeth 132 on the other side of arm 120 to disengage from the matching ratchet teeth 133 on the articulation drive shaft 122, which is connected to the sliding base 60 pulling it and strips 72 proximally. This allows drive shaft 122 to move along the axis of the instrument and accomplish articulation of the tip.

While the ratchet is disengaged by depressing the release trigger, the surgeon can then either depress the actuator 22 to articulate the tip or release the lever allowing the actuator reset spring 121 to push the actuator back up, and thereby de-articulate the end effector. When the surgeon sees that the end effector is at the desired articulation angle, he can then release the actuator 22, which will move back forward due to the force supplied by the release trigger reset spring 123. Correspondingly, pall arm reset spring 125 will then cause the ratchet pall arm 120 to rotate back so that the ratchet teeth 132 will then re-engage the teeth 133 on the articulation drive shaft 122, thereby preventing any change in the setting of the articulation mechanism. This ratchet arrangement is designed so as to minimize the risk of any unintended changes in the articulation of the instrument tip. This is accomplished by having the surgeon holding the release trigger 24 in the depressed position while changing the tip articulation angle. The instrument articulation cannot be readily changed without the depression of this trigger.

The actuator also includes a means for preventing the first strip from moving proximally beyond a predetermined amount and thereby causing the end effector to bend only up to a predetermined angle. This is done by limiting the movement of the drive shaft 122, by correctly sizing the length of the teeth 133. A preferred maximum angle for end effector articulation is 90 degrees. In addition, the predetermined angle is such that the links never make contact with one another. Figure 7 shows the instrument in its articulated position.

Lastly, it is preferred that device 10 be sterilized. This can be done by any number of ways known to those skilled in the art including beta or gamma radiation, ethylene oxide, steam.

It will become readily apparent to those skilled in the art that the above invention has equally applicability to other types of implantable bands. For example, bands are used for the treatment of fecal incontinence. One such band is described in U.S. Patent 6,461,292 . Bands can also be used to treat urinary incontinence. Bands can also be used to treat heartburn and/or acid reflux. One such band is described in U.S. Patent 6,470,892. Bands can also be used to treat impotence.

While preferred embodiments of the present invention have been shown and described herein, it will be obvious to those skilled in the art that such embodiments are provided by way of example only. Numerous variations, changes, and substitutions will now occur to those skilled in the art without departing from the invention. For example, as would be apparent to those skilled in the art, the disclosures herein have equal application in robotic-assisted surgery. In addition, it should be understood that every structure described above has a function and such structure can be referred to as a means for performing that function. Accordingly, it is intended that the invention be limited only by the scope of the appended claims.

## Claims

1. A device (10) for aiding a surgical procedure, said device comprising:
a. an clongated shaft (12) having a distal end (14), a proximal end (16) and a longitudinal axis (18) therebetween, said proximal end having a handle (20) attached thereto, and said distal end having an end effector (50) attached thereto;
b. said shaft having a first strip (72) running along said longitudinal axis from said handle to a distal end of said end effector where it is attached thereto, and a second strip (74), parallel to said first strip, running along said longitudinal axis from said distal end of said shaft to said distal end of said end effector where it is attached thereto;
c. said end effector comprising a plurality of spaced apart links (52) disposed along said longitudinal axis between said distal end of the end effector said distal end of said shaft such that said strips run through said links, and each link is attached to at least one strip;
d. an actuator (22) attached to said handle, said actuator pulls said first strip proximally, causing said links and said end effector to curve in the direction of said first strip, said actuator including a means for preventing said first strip from moving proximally beyond a predetermines amount and thereby causing said end effector to bend only up to a predetermined angle, wherein said predetermined angle is such that said links never make contact with one another.

2. The device of claim 1, wherein said first strip comprises a plurality of juxtaposed strips.

3. The device of claim 1 or claim 2, wherein said second strip comprises a plurality of juxtaposed strips.

4. The device of any one of claims 1 4, wherein each of said links is attached to said second strip.

5. The device of claim any one of claims 1-5 wherein said links have internal concave surface adjacent said second strip.

6. The device of any one of claims 1-5 wherein said links have an internal convex (53) surface adjacent said first strip.

7. The device of any one of claims 1-6, wherein said predetermined angle is no greater than 90 degrees.

8. The device of any one of claims 1 7 wherein said end effector is covered with an elastomeric hoot (90).

9. The device of claim 8 wherein said boot is made from pellethane.

10. The device of any one of claims 1-9), wherein said first and second strips are made from stainless Steel.

## Patentansprüche

1. Vorrichtung zum Unterstützen einer chirurgischen Prozedur, umfassend:
a. einen langgestreckten Schaft (12) mit einem Distalende (14), einem Proximalende (16) und einer Längsachse (18) dazwischen, wobei das Proximalende einen Griff (20) aufweist, der daran angebracht ist, und das Distalende einen Endeffektor (50) aufweist, der daran angebracht ist;
b. wobei der Schaft einen ersten schmalen Streifen (72), der entlang der Längsachse von dem Griff zu einem Distalende des Endeffektors verläuft, an dem er angebracht ist, und einen zum ersten schmalen Streifen parallelen zweiten schmalen Streifen (74) aufweist, der entlang der Längsachse von dem Distalende des Schafts zu dem Distalende des Endeffektors verläuft, an dem er angebracht ist;
c. wobei der Endeffektor mehrere in einem Abstand zueinander angeordnete Verbindungsglieder (52) umfasst, die entlang der Längsachse zwischen dem Distalende des Endeffektors dem Distalende des Schafts so angebracht sind, dass die Streifen durch die Verbindungsglieder hindurch verlaufen, und jedes Verbindungsglied an wenigstens einem schmalen Streifen angebracht ist;
d. ein an dem Griff angebrachtes Betätigungsglied (22), das den schmalen Streifen in Proximalrichtung zieht, wodurch sich die Glieder und der Endeffektor in Richtung des ersten Streifens krümmen, wobei das Betätigungsglied eine Einrichtung zum Verhindern einer proximalen Bewegung des ersten schmalen Streifens über einen vorbestimmten Umfang hinaus umfasst, wodurch der Endeffektor nur bis zu einem vorbestimmten Winkel gebogen wird, wobei der vorbestimmte Winkel so eingestellt ist, dass die Glieder nie in Kontakt miteinander kommen.

2. Die Vorrichtung nach Anspruch 1, wobei der erste schmale Streifen mehrere nebeneinander angeordnete, schmale Streifen umfasst.

3. Die Vorrichtung nach Anspruch 1 oder 2, wobei der zweite schmale Streifen mehrere nebeneinander angeordnete, schmale Streifen umfasst.

4. Die Vorrichtung nach einem der Ansprüche 1 bis 4, wobei jedes der Verbindungsglieder an dem zweiten schmalen Streifen angebracht ist.

5. Die Vorrichtung nach einem der Ansprüche 1 bis 5, wobei die Verbindungsglieder eine an den zweiten schmalen Streifen angrenzende, interne konkave Fläche aufweisen.

6. Die Vorrichtung nach einem der Ansprüche 1 bis 5, wobei die Verbindungsglieder eine an den ersten schmalen Streifen angrenzende, interne konvexe Fläche (53) aufweisen.

7. Die Vorrichtung nach einem der Ansprüche 1 bis 6, wobei der vorbestimmte Winkel nicht größer als 90° ist.

8. Die Vorrichtung nach einem der Ansprüche 1 bis 7, wobei der Endeffektor mit einer Elastomermanschette (90) bedeckt ist.

9. Die Vorrichtung nach Anspruch 8, wobei die Elastomermanschette aus Pellethan gefertigt ist.

10. Die Vorrichtung nach einem der Ansprüche 1 bis 9, wobei der erste und zweite schmale Streifen aus rostfreiem Stahl gefertigt sind.

## Revendications

1. Dispositif (10) d'aide pour une intervention chirurgicale, ledit dispositif comprenant :
a. une tige allongée (12) ayant une extrémité distale (14), une extrémité proximale (16) et un axe longitudinal (18) entre elles, ladite extrémité proximale ayant une poignée (20) fixée à celle-ci, et ladite extrémité distale ayant un effecteur terminal (50) fixé à celle-ci ;
b. ladite tige ayant une première bande (72) s'étendant le long dudit axe longitudinal de ladite poignée à une extrémité distale dudit effecteur terminal lorsqu'il est fixé à celle-ci, et une seconde bande (74) parallèle à ladite première bande, s'étendant le long dudit axe longitudinal de ladite extrémité distale de ladite tige jusqu'à ladite extrémité distale dudit effecteur terminal lorsqu'il est fixé à celle-ci ;
c. ledit effecteur terminal comprenant une pluralité de liaisons espacées. (52) disposées le long dudit axe longitudinal entre ladite extrémité distale de l'effecteur terminal et ladite extrémité distale de ladite tige, de sorte que lesdites bandes s'étendent à travers lesdites liaisons, et chaque liaison est fixée à au moins une bande ;
d. un actionneur (22) fixé à ladite poignée, ledit actionneur tire ladite première bande de manière proximale, amenant lesdites liaisons et ledit effecteur terminal à s'incurver dans la direction de ladite première bande, ledit actionneur comprenant des moyens pour empêcher ladite première bande de se déplacer de manière proximale au-delà d'une quantité prédéterminée et amenant ainsi ledit effecteur terminal à fléchir uniquement jusqu'à un angle prédéterminé, dans lequel ledit angle prédéterminé est tel que lesdites liaisons ne sont jamais en contact les unes avec les autres.

2. Dispositif selon la revendication 1, dans lequel ladite première bande comprend une pluralité de bandes juxtaposées.

3. Dispositif selon la revendication 1 ou la revendication 2, dans lequel ladite seconde bande comprend une pluralité de bandes juxtaposées.

4. Dispositif selon l'une quelconque des revendications 1 à 4, dans lequel chacune desdites liaisons est fixée sur ladite seconde bande.

5. Dispositif selon l'une quelconque des revendications 1 à 5, dans lequel lesdites liaisons ont une surface concave interne adjacente à ladite seconde bande.

6. Dispositif selon l'une quelconque des revendications 1 à 5, dans lequel lesdites liaisons ont une surface interne convexe (53) adjacente à ladite première bande.

7. Dispositif selon l'une quelconque des revendications 1 à 6, dans lequel ledit angle prédéterminé n'est pas supérieur à 90 degrés.

8. Dispositif selon l'une quelconque des revendications 1 à 7, dans lequel ledit effecteur terminal est recouvert avec une botte élastomère (90).

9. Dispositif selon la revendication 8, dans lequel ladite botte est réalisée à partir de Pellethane.

10. Dispositif selon l'une quelconque des revendications 1 à 9, dans lequel lesdites première et seconde bandes sont réalisées à partir d'acier inoxydable.
